# EUROPEAN PATENT SPECIFICATION

(11) **EP 4 046 573 B1**
(45) Date of publication and mention of the grant of the patent: **06.09.2023**
(21) Application number: 22154296.2
(22) Date of filing: 31.01.2022
(51) Int. Cl.: A61B 5/257, A61B 5/273, A61B 5/282

(54) **ELECTRODE SYSTEM FOR MULTIPLE ACQUISITION OF ELECTROCARDIOGRAPHIC DERIVATIONS**
ELEKTRODENSYSTEM ZUR MEHRFACHEN ERFASSUNG ELEKTROKARDIOGRAPHISCHER ABLEITUNGEN
SYSTEME D'ELECTRODE POUR L'ACQUISITION MULTIPLE DE DERIVATIONS ELECTROCARDIOGRAPHIQUES

(30) Priority: 03.02.2021 IT 202100002276
(43) Date of publication of application: 24.08.2022
(73) Proprietor: Praxe Srl, 60121 Ancona (IT)
(72) Inventor: FRANCO, Antonio, 60121 ANCONA (IT); SCALISE, Lorenzo, 60121 ANCONA (IT); CASOLI, Gloria, 60121 ANCONA (IT)
(74) Representative: Cutropia, Gianluigi

(56) References cited:
- EP-A1- 2 105 089
- WO-A1-2018/157044
- CN-U- 201 537 089
- US-B1- 7 266 405

## Description

The present patent application for industrial invention relates to an electrode system for the multiple acquisition of electrocardiographic derivations.

In particular, the present invention relates to an electrode system for the multiple acquisition of electrocardiographic derivations used for performing an electrocardiographic examination on human and animal subjects.

It is known that an electrocardiograph is a diagnostic tool capable of recording the electrical potentials related to the cardiac activity by means of external electrodes connected on the thoracic and/or dorsal portion, possibly also on the wrists and on the ankles.

The potentials that are generated on the surface of the body are detected in certain universally accepted positions in order to have comparable graphs (derivations). The cardiac signal generates potential differences on the surface of the body which are detected with special electrodes.

The potentials on the skin surface are not strictly correlated to the real cardiac potentials, but a good degree of correlation can be obtained by representing such an electrical activity as a product of the motion of a dipole whose moment is characterized by parameters (intensity and direction) that vary over time.

Each one of said derivations corresponds to a characteristic recording traces; ECGs can report and/or display one, three, six or twelve traces simultaneously, and the choice of the derivation to be recorded can be made by means of a manual, electronic or automatic sequential switch. The electrical signal detected on the surface of the body has a relatively small amplitude (of the order of 1 mV), and therefore the magnitude (gain) and the quality of the amplification are particularly important. The cardiac signal is picked up by the electrodes that are applied to the skin to detect the signal; they can be reusable or disposable. The reusable electrodes are made of steel (plates, generally used in the derivations on the limbs, or suction cups, used in the so-called precordial derivations on the chest) or silver/silver chloride: although more expensive, the latter are more delicate, are mounted on self-adhesive supports, which are usually very flexible, and are attached to the skin duly depilated and degreased.

The electrodes, in variable number and properly arranged on the body of the patient, are connected to the ECG apparatus by means of the so-called patient cable or multi-conductor cable consisting of a set of separate very flexible electrical wires. The cardiac signal coming from the electrodes is amplified by a preamplifier at the input of which a system of filters eliminates the continuous component and determines the minimum recordable frequency. The signal picked up by the electrodes is checked with a fault detector, and successively enters the protection circuit whose task is to avoid that a too high voltage, such as that which occurs in the case of defibrillation, reaches the amplification circuits, damaging them. Then, a derivation selector is provided with the task of suitably combining the signals coming from the electrodes connected to the patient in such a way that the operator can select the various derivations without having to intervene on the positioning of the electrodes. In the case of derivations on the frontal plane, the electrodes must be positioned on the right arm (Right Arm, RA), left arm (Left Arm, LA), left leg (Left Leg, LL), whereas in the case of derivations on the transverse plane, six electrodes are positioned over an arc of 90 degrees on the left side of the chest, and the average voltage of RA-LA-LL is used as a reference for the potentials. By means of a suitable control, the calibrator allows the operator to manually introduce a signal of known and constant amplitude for calibrating the instrument. The signal, which is applied at the beginning of the amplification chain, is typically of the order of few mV. The preamplifier is used to realize a first amplification of the ECG signal, without distorting it, and at the same time to minimize interferences and artifacts. The position of the electrode deeply affects the type of detection obtained. To understand how to position the electrodes in optimal position, it is necessary to define the difference between a linear conductor, which has the same voltage at all points along its length, and a conductor volume, in which the voltage can vary considerably in different areas. By way of example, the thorax behaves as a conductor volume, and therefore the precise position of the electrodes on the thorax is essential for a correct recording. On the contrary, the limbs behave as linear conductors and therefore they can be considered as extensions of the cables of the electrodes.

The reusable clamp electrodes and the suction cup electrodes are used on multiple patients and allow for rapidly performing a basic electrocardiographic analysis on stationary, typically lying patients. Instead, the disposable adhesive electrodes are used for prolonged examinations or for examinations in dynamic conditions and to obtain more precise signals.

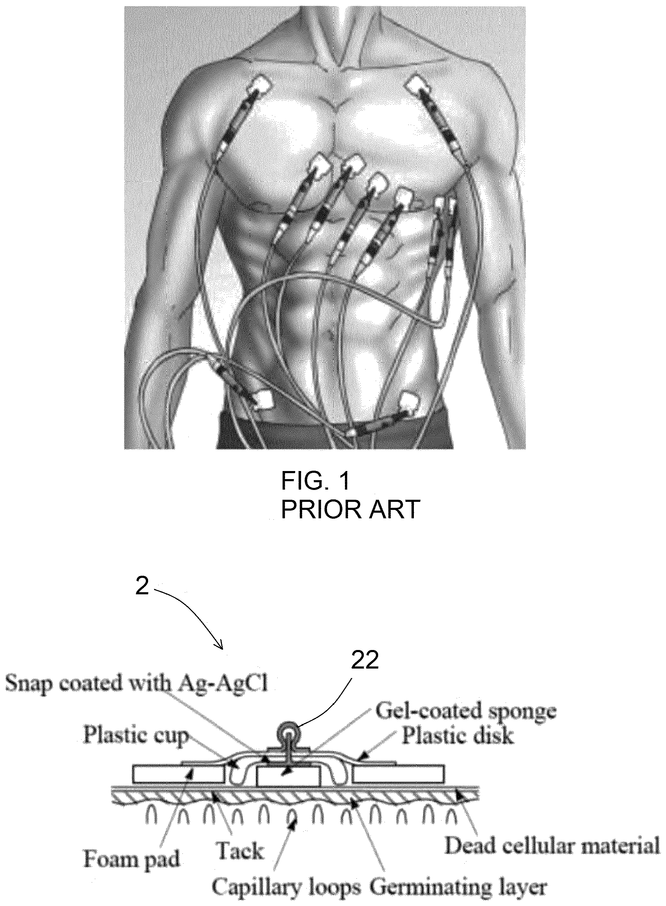

The major limitation of the disposable electrodes for ECG of the prior art is related to the fact that the operator who is performing the electrocardiographic examination must position up to ten electrodes on the thoracic portion of the patient and then each electrode must be connected with a single conductor cable to the electrocardiographic measurement system by means of the patient cable. As a result, the operation is particularly time-consuming and does not allow the patient to move easily due to the presence of the electrodes and the conductor cables. Fig. 1 (Prior Art) shows an example of how the electrodes should be applied to the patient's body.

An example of an electrode system for ECG is disclosed in the patent application US6415169B1 which discloses a flexible multiple electrode assembly that includes at least one fixed electrode; at least one extendible electrode; and electrically conductive interconnections coupling the at least one fixed electrode and the at least one extendible electrode to a common connector. The at least one extendible electrode is adapted to be physically separable from the at least one fixed electrode while remaining electrically coupled to the common connector. In one embodiment, an array of fixed and extendible electrodes is configured for the acquisition of electrical pulses from a heart for transmission to an electrocardiograph (EKG or ECG) device. Such a solution provides for physically separating the at least one extendible electrode from the at least one fixed electrode along the entire length of the conductor cable in order to free it, thus making the preparation and application process of the electrodes time-consuming and complicated. Moreover, the solution described for the storing of the conductor cables provides for freeing only sections with fixed length of the cable. Furthermore, it must be noted that, once all of the electrodes have been freed, the risk of erroneously positioning one or more electrodes cannot be avoided.

Another example of an electrode system for ECG is disclosed in the patent application US2010076295A1 which discloses an electrode set comprising a base comprising an insulating material and a plurality of electrodes disposed on the base. The electrode set also comprises a plurality of conductors disposed on the base. The conductors comprise a generally insulated conductive material, each of said plurality of conductors being connected to one of said plurality of electrodes. The electrodes are positioned on the base relative to each other such that they can be collectively placed on specific portions of a patient's anatomy. Also for such a solution it is necessary to free the conductor for its entire length before positioning the electrodes; moreover it must be noted that some of the electrodes are not separable from the others. Furthermore, the distance between the various electrodes is fixed, it being predetermined and not modifiable, either with respect to the other electrodes or to the patient's morphological configuration.

US2105089A1 discloses an electrode assembly for ECG, comprising a flexible support whereon a plurality of electrodes, at least one terminal for the electrical connection to an ECG device and a plurality of conductive tracks connecting the electrodes to the electrical connection terminal are provided. The support comprises elastically deformable portions disposed between adjacent electrodes to bring the assembly from a planar configuration to a three-dimensional configuration.

The elastically deformable portions are obtained by means of cuts that extend for the entire length of the deformable portion. The cuts are made in such a way that when the portions are extended, strips with a double spiral structure are generated. At least one conductive track is printed on each strip. The electrode assembly disclosed in US2105089A1 is impaired by several drawbacks.

A first drawback concerns the fact that, when they are stretched, the deformable portions always tend to return to their original shape due to the retention force generated by the spiral structures. Therefore, during use, it is possible that the electrodes detach from the patient's skin precisely because of the elastic return action of said deformable portions.

A second drawback arises from the fact that, when they are stretched, the elastically deformable portions generate conductive coils that behave as radiant elements (antennas) that can generate and pick up electromagnetic noise from the surrounding environment, therefore altering the useful signal represented by the electrical potential detected by the electrodes.

Moreover, using the assembly described in US2105089A1 an operator is not free to position the electrodes in the positions of the patient's body that are considered to be most appropriate, since the positioning of said electrodes is constrained by the geometry of the structure and the maximum length that can be reached by the elastically deformable portions. It should also be noted that the electrode assembly is quite expensive and complex to make.

WO2018157044A1 discloses an electrode placement device, comprising a body composed of a plurality of flexible extension members, wherein electrodes and cables are disposed. The placement device is very cumbersome and, when extended, the electrodes may be detached from the patient's skin due to the elastic return action of said flexible extension members.

US7266405B1 discloses a reusable and portable ECG signaling device, comprising a thin, flexible electrode support supporting a plurality of electrodes. The electrodes are ten in number, namely eight electrodes in fixed position and two electrodes of extendible kind because of serpentine strips supporting the two extendible electrodes. The device can be folded when is not used. The ten fixed electrodes cannot be separated or moved apart relative to each other. Moreover, also in such a case, the two extendible electrodes can be detached from the patient's skin because of the elastic return of the elastic serpentine strips.

EP2105089A1 discloses an electrode assembly for electrocardiogram, comprising a flexible support on which a plurality of electrodes are disposed, at least one terminal for electrical connection to an electrocardiogram instrument and a plurality of conductive tracks that connect the electrodes to the electrical connection terminal.

The purpose of the present invention is to eliminate the drawbacks of the prior art by providing an electrode system for the multiple acquisition of electrocardiographic derivations that makes the installation operation easy and practical, reduces or eliminates the presence of the electrical conductors connecting the individual electrodes to the electrocardiographic system and allows the connection of a portable ECG device directly to the electrode system, eliminating the need for using the patient cable or multi-conductor, therefore having suitable characteristics to overcome the limitations that have been previously highlighted, which are typical of the current electrode systems for EGC.

A further purpose of the present invention is to devise an electrode system that is efficient, safe and easy to realize.

Another purpose of the present invention is to devise an electrode system that has a minimal footprint prior to use and during use allows an operator to apply the electrodes anywhere in a stable manner without the risk that such electrodes may become detached from the patient's skin during use.

These purposes are achieved in accordance with the invention with the features of the appended independent claims.

Advantageous embodiments appear from the dependent claims.

The electrode system according to the invention is defined by claim 1.

For the sake of explanatory clarity, the description of the electrode system according to the invention is continued with reference to the appended drawings, which are of illustrative and non-limiting value only, wherein:
- Fig. 1 (Prior Art) is a schematic view of the arrangement of electrodes for a standard electrocardiographic examination with fundamental, augmented and precordial derivations, according to the prior art;
- Fig. 2 is a schematic sectional vies of a disposable electrode;
- Figs. 3 and 4 are schematic top views of an electrode system according to the invention in space-saving configuration; in Fig. 3 and in Fig. 4 the electrode system has respectively one and two multi-pole terminals for the electrical connection to a signal reception and processing system;
- Fig. 5 is a schematic sectional view of an electrode and a fastening body to which the electrode is fastened;
- Figs. 6 and 6A are a schematic side view illustrating a first embodiment of a support for a connector cable; Fig. 6 shows the support that holds two electrodes connected; Fig. 6A shows the separate support that allows for the unrolling of the connector cable and the reciprocal removal of the two electrodes;
- Figs. 7 and 7A show a second embodiment of a support for a connector cable; Fig. 7 shows the support that holds two electrodes connected; Fig. 7A shows the separate support that allows for the unrolling of the connector cable and the reciprocal removal of the two electrodes;
- Figs. 8 and 8A show a third embodiment of a support for a connector cable; Fig. 8 shows the support that holds two electrodes connected; Fig. 8A shows the separate support that allows for the unrolling of the connector cable and the reciprocal removal of the two electrodes;
- Fig. 9 is a schematic view of the electrode system before use in a first embodiment;
- Fig. 9A shows the electrode system of Fig. 9 in use;
- Fig. 10 is a schematic view of the electrode system before use in a second embodiment;
- Fig. 10A is a schematic view of the electrode system of Fig. 10 in use;
- Figs. 11, 12 and 13 are schematic views of three different electrode systems, prior to use, in a third embodiment;
- Fig. 14 is a schematic view of the electrode system of Fig. 13 in use;
- Figs. 15 and 16 are a schematic view of a non-portable ECG measurement instrument and of a portable ECG measurement instrument comprising the electrode system according to the invention, respectively.

With reference to Figs. 2 to 14, an electrode system for the multiple acquisition of electrocardiographic derivations is described, which is generally indicated with reference numbers 100, 200 and 300.

In particular, the system of the present invention is indicated with three different numbers because three different embodiments will be described, which differ in particular in the configuration of the system and in the relative position of electrodes of the electrode system.

All three embodiments of the electrode system (100, 200, 300) comprise:
- a plurality of electrodes (2) of adhesive disposable type, suitable for being applied on a user's body;
- at least one electrical connection terminal (1) for connecting the electrode system (100, 200 , 300) with a signal receiving and processing system;
- a plurality of connector cables (3) connecting each electrode (2) to the at least one electrical connection terminal (1); and
- a plurality of supports (4) that connect said electrodes (2) to each other, each support (4) supporting at least one connector cable (3) that is rolled or bent in serpentine configuration; wherein each support (4) keeps the two adjacent electrodes (2) proximate to each other in a space-saving configuration of the electrode system prior to use.

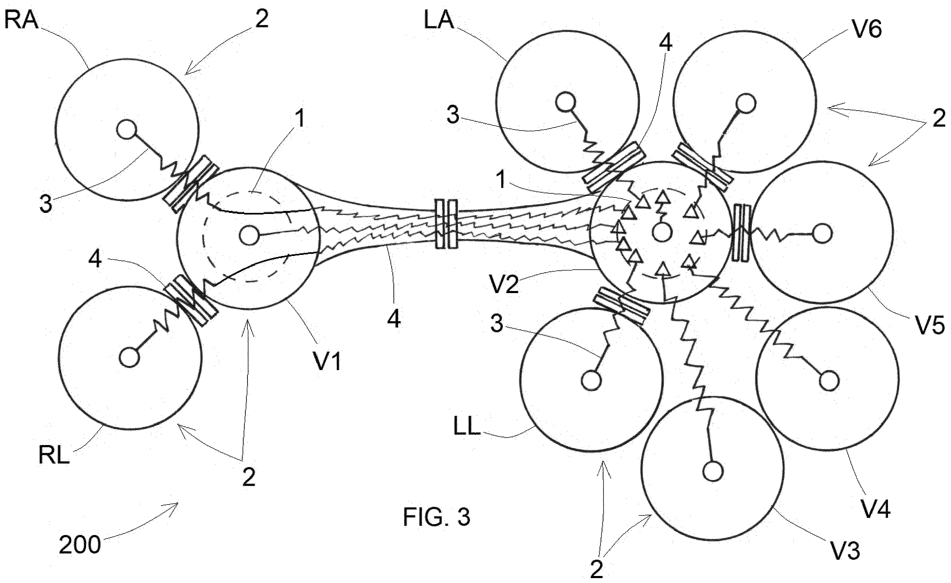

Otherwise said, prior to the use of the electrode system (100, 200, 300), the supports (4) keep all electrodes (2) close to each other so that the electrode system has a minimum footprint (see Figs. 3, 4, 9, 10, 11, 12 and 13) to allow for easy storage and/or transportation.

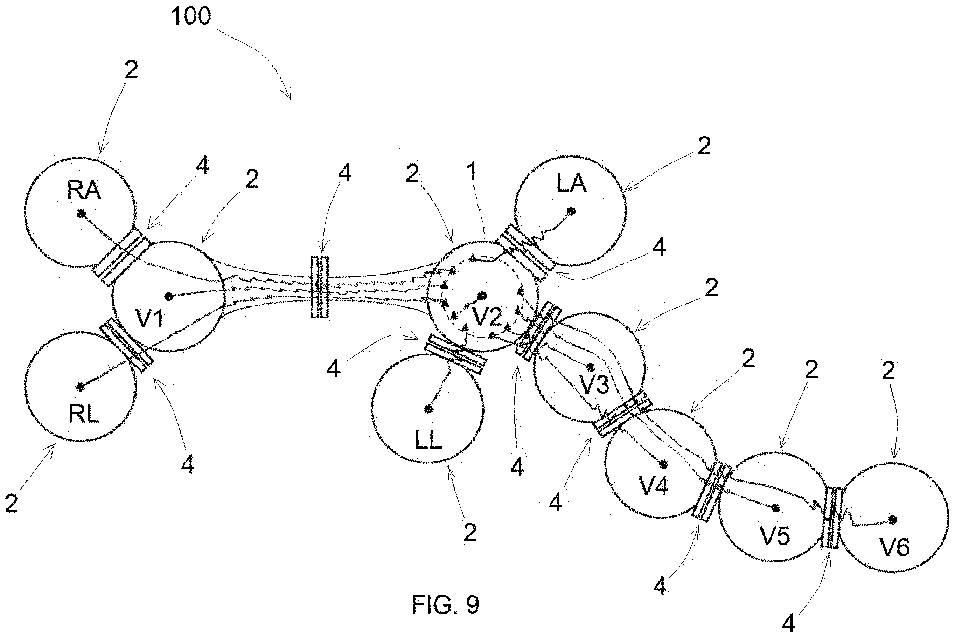

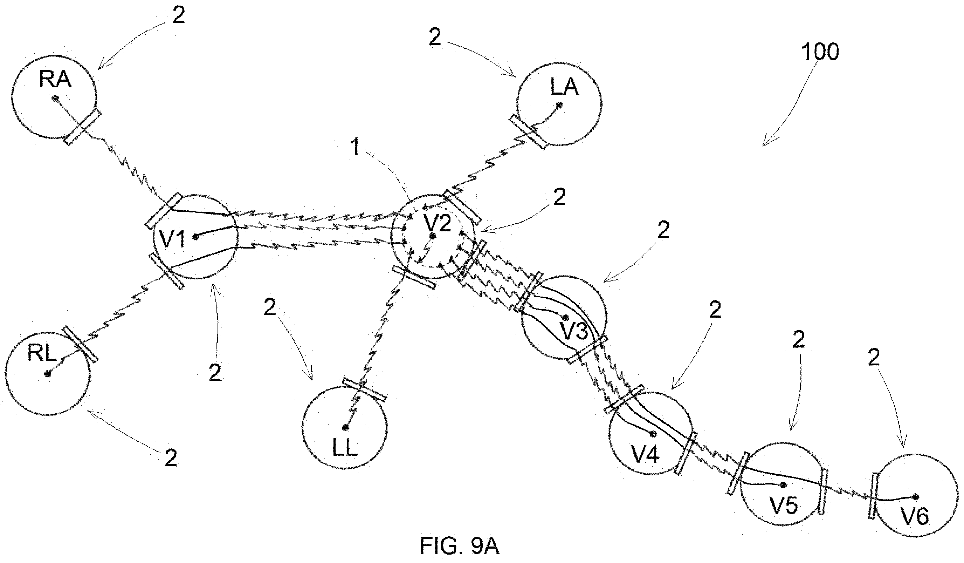

The peculiarity of the electrode system (100, 200, 300) according to the invention is that each support (4) is of removable or separable type, in such a way that, once the support (4) is removed or separated, the two electrodes (2) connected to the support (4) can be moved away from each other and the connector cables (3) can be unrolled and extended in order to use the system (see Figs. 9A, 10A, 14).

Otherwise said, in order to use the system it is necessary to remove one, some, or all of the supports (4) and position the various electrodes (2) in the most suitable parts of the patient's body.

The fact that the supports (4) are removable or separable, allows to completely free the connector cables (3), in such a way that the operator can position the electrodes (2) in any desired position without any structural constraints.

Therefore, thanks to the electrode system (100, 200, 300), the connector cables (3) can be extended in any direction and the electrodes can be positioned in any part of the patient's body.

As mentioned above, the electrodes (2) are of the disposable type.

An electrode (2) of disposable type is shown for illustrative purposes in Fig. 2.

The electrode (2) comprises:
- a disk of metallic material suitable for being disposed in contact with the user's skin;
- a connector (22) consisting of a connection clip that electrically connects the electrode (2) to the corresponding connector cable (3);
- a disk of adhesive material that keeps the electrode (2) firmly attached to the patient's skin.

The disk of metallic material is usually made of silver chloride. In addition, such a metallic disk is preferably pre-impregnated with a conductive gel with a base of water and ionic salts that allow a better efficiency in collecting the electric potential.

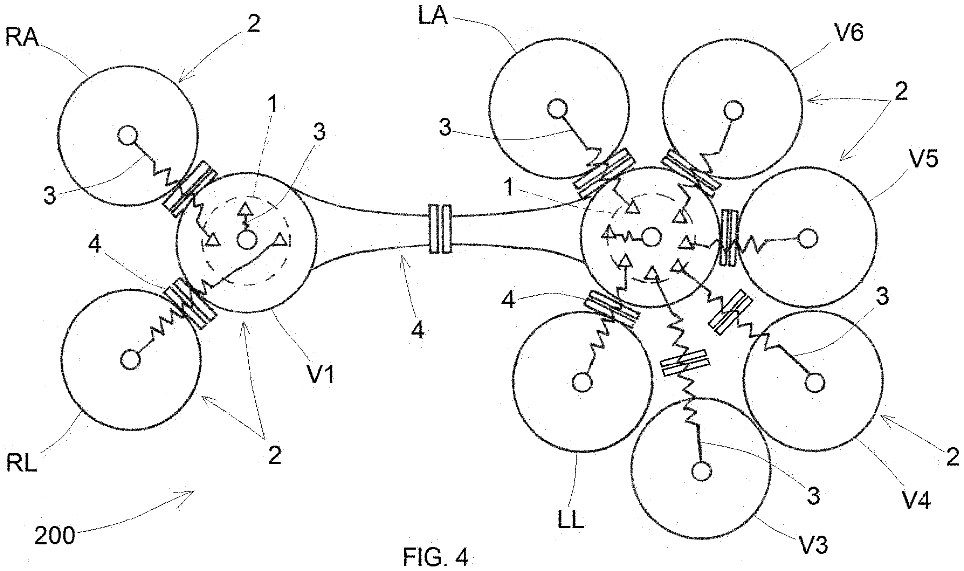

With reference to Figs. 3 and 4, the configuration of the connector cables (3) of the system according to invention (3) is shown schematically.

Each connector cable (3) comprises a first end, (indicated with a solid circle in Figs. 3 and 4) that is electrically connected to the connector (22) of the corresponding electrode (2), and a second end (indicated with a triangle in Figs. 3 and 4) that is electrically connected to a corresponding connector obtained in the at least one electrical connection terminal (1).

Again with reference to Figs. 3 and 4, said at least one electrical connection terminal (1) may comprise:
- a single electrical connection terminal (1) positioned in correspondence with one (V2) of said electrodes (2); or
- two electrical connection terminals (1) positioned in correspondence with two (V1 and V2) of said electrodes (2).

If the system has only one electrical connection terminal (1) then all connector cables (3) terminate in correspondence with the electrical connection terminal (1) (see Fig. 3); if the system has two electrical connection terminals (1) then some cables terminate in correspondence with one of the two electrical connection terminals (1) and the other connector cables (3) terminate in correspondence with the other electrical connection terminal (1). In Figs. 3 and 4 the electrical connection terminal (1) or the electrical connection terminals (1) are shown schematically with a dotted circle.

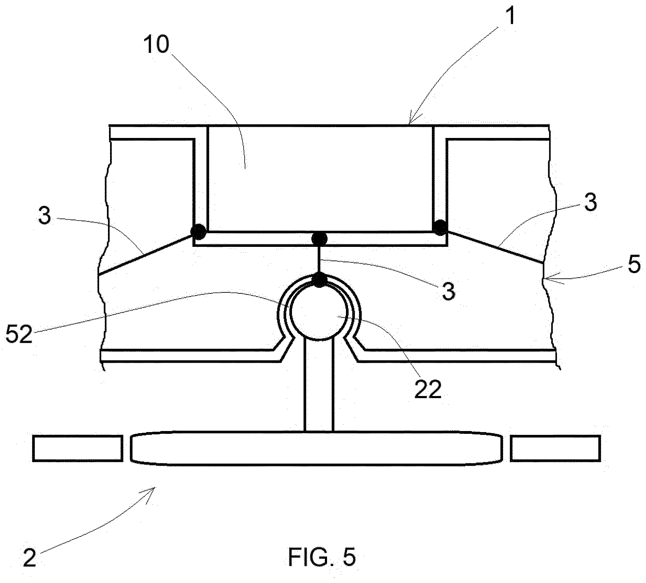

With reference to Fig. 5, preferably the electrical connection terminal (1) consists of an attachment pad (10) whereon said connectors of the second ends of the connector cables (3) are provided.

The attachment pad (10) is suitable for being attached to:
- a portable device housing said signal receiving and processing system, or
- a connector of a patient cable connected to the signal reception and processing system mounted on an ECG device separate from the patient.

The electrical potentials detected by the electrodes (2) arrive at the electrical connection terminal (1) via the connector cables (3). By means of the connection of the signal receiving and processing system to the electrode system (100, 200, 300), said electrical potentials can be processed in order to obtain information on the patient's cardiac activity.

With reference to Figs. 5 to 8A, for each electrode (2) the electrode system (100, 200, 300) comprises a coupling body (5) connected to at least one support (4) and comprising a connector (52) coupled with the connector (22) of the electrode (2). The connector cable (3) is electrically connected to the connector (52) of the coupling body (5).

The connector (52) of the coupling body (5) comprises a housing that is suitably shaped to engage with said connection clip of the connector (61) of the electrode (2). Preferably, the coupling body (5) is box-shaped and made of plastic material.

Referring only to Fig. 5, said attachment pad (10) of the electrical connection terminal (1) is provided on the coupling body (5).

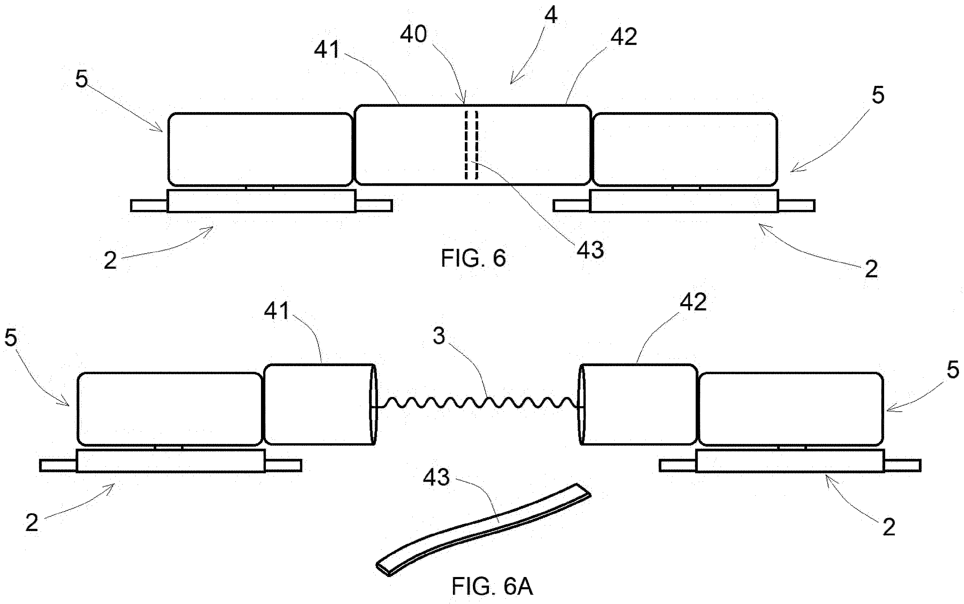

With particular reference to Figs. 6, 6A, 7, 7A, 8 and 8A, different embodiments of the support (4) are shown.

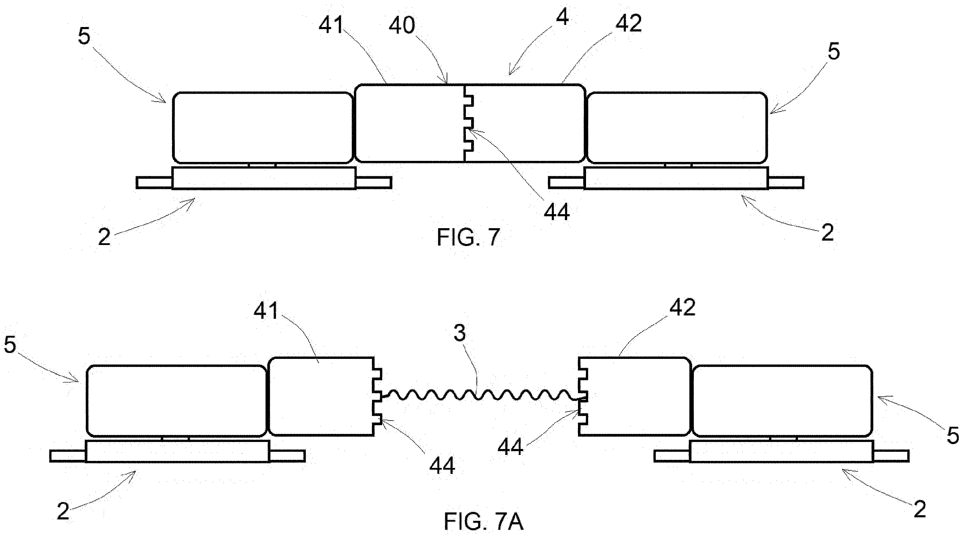

In the embodiment shown in Figs. 6, 6A, 7, and 7A said support (4) comprises a casing (40) which accommodates the connector cable (3) that is rolled or bent in serpentine configuration.

The casing (40) comprises a first half-shell (41) and a second half-shell (42) that are separable from each other.

The casing (40) comprises separable connection means that are interposed between the two half-shells (41, 42) and that allow the two half-shells (41, 42) to be separated from each other so that the casing (40) can be opened and the connector cable (3) disposed therein can be unrolled or extended.

With reference to Figs. 6 and 6A, said separable connection means comprise a removable clamp (43) connected to the two half-shells (41, 42) by means of pre-cut lines. In such a case, in order to separate the two half-shells (41, 42), the user will have to grasp the removable clamp (43) and pull it away with a force sufficient to overcome the resistance provided by the pre-cut lines.

With reference to Figs. 7 and 7A, the separable connection means comprise interlocking means (44), such as teeth and seats, respectively provided on one half-shell (41) and on the other half-shell (42).

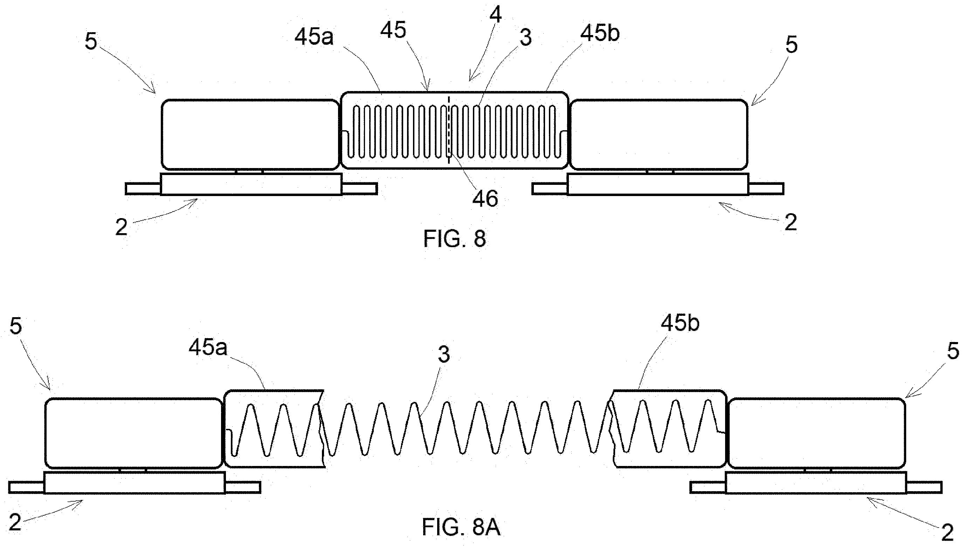

In the embodiment shown in Figs. 8 and 8A, said support (4) comprises a flat two-dimensional element (45) that extends between the two electrodes and has an adhesive surface whereon the connector cable (3), which is rolled or bent in serpentine configuration, is attached.

In such a case, the two-dimensional element may comprise two separable portions (45a, 45b) connected by means of a pre-cut line (46).

In order to separate the two separable portions (45a, 45b), it will be necessary to grasp the two separable portions (45a, 45b) and pull them in opposite directions until they are detached from each other at the pre-cut line (46).

Alternatively, although not shown in the accompanying figures, the two-dimensional element (45) may also have one end attached to one electrode (i.e. to the coupling body (5)) and the other end to the other electrode. In such a case, in order to disconnect the two-dimensional element (45) it will be necessary to pull it off completely in the same way as a plaster.

Therefore, prior to use, the coupling bodies (5) and the supports (4) define a compact structure that is easy to store and space-saving. By removing or separating the supports (4), the structure can be divided into the different coupling bodies (5), thus allowing the electrodes (2) - which are coupled to the coupling bodies (5) - to be positioned in any position on the patient's body.

When the electrode system (100, 200, 300) is associated with said signal receiving and processing system, a real ECG measurement instrument is realized.

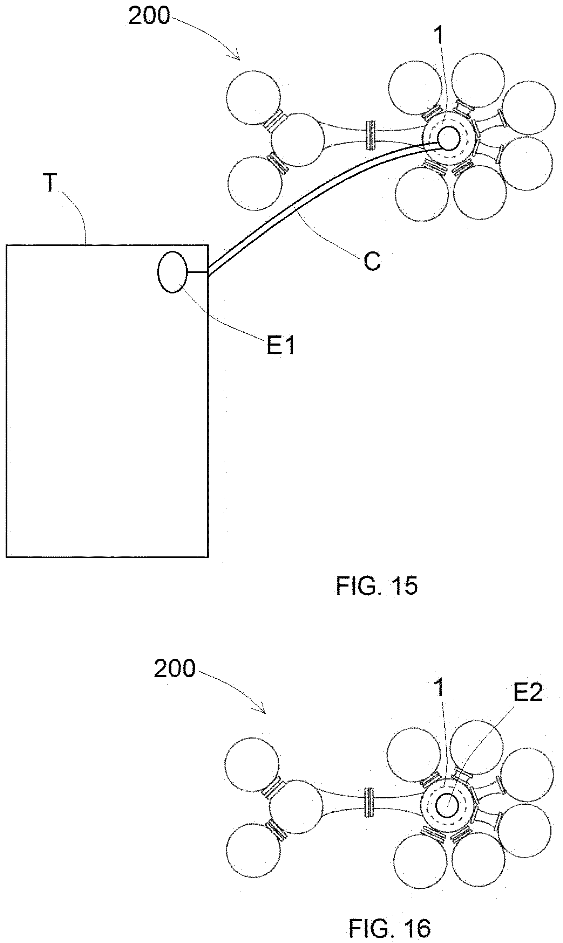

The ECG measurement instrument can be of non-portable or portable type, as shown in Fig. 15 and Fig. 16 respectively.

In the non-portable ECG measurement instrument shown in Fig. 15, the signal receiving and processing system comprises:
- a computing device (E1) mounted on an ECG apparatus (T) separate from the patient;
- a patient cable (C) that electrically connects the at least one connection terminal (1) to the computing device (E1).

On the other hand, in the portable ECG measurement instrument shown in Fig. 16, the electronic signal receiving and processing system can be housed in a single device (E2) (if the electrode system comprises only one connection terminal (1)) directly connected and coupled to the connection terminal (1), or in two devices (if the electrode system comprises two connection terminals (1)) directly connected and coupled to the two connection terminals (1). In turn, the two devices are in communication with each other by means of a cable that electrically connects the two connection terminals, or by means of antennas of the two devices that are coupled together.

In the portable ECG version, the use of the patient cable or the multi-conductor cable is avoided.

The portable ECG measurement instrument of Fig. 16 is particularly suitable for a Holter type ECG monitoring. The electrode system with the signal receiving and processing system (consisting of one or two devices) is extremely lightweight and does not hinder the daily movements made by a user during an ordinary day.

Now, with reference to Figs. 9 to 14A , the three possible embodiments of the system (100, 200, 300) will be illustrated.

In particular, the three versions differ in the position and/or in the number of electrodes (2) of which the system is composed (100, 200, 300).

It should be noted that all the features of the elements described above can be applied for all three embodiments of the electrode system (100, 200, 300).

Referring to Figs. 9 and 9A, the electrode system (100) comprises ten electrodes (2) and ten conductor cables (3).

In particular, the electrode system (100) comprises two central electrodes (V1, V2), namely a first central electrode (V1) and a second central electrode (V2). As shown in Fig. 9A, the connection terminal (1) is positioned in correspondence with the second central electrode (V2). It should be noted, however, that nothing would change for the achievement of the objectives pursued by the invention, if the connection terminal (1) were positioned only in the first central electrode (V1) or if the electrode system (100) comprised two connection terminals positioned in the two central electrodes (V1 and V2).

In the electrode system (100) of the first embodiment, the ten electrodes comprise eight external electrodes, namely:
- a first electrode (RA) connected to the first central electrode (V1) by means of one of said supports (4);
- a second electrode (RL) connected to the first central electrode (V1) by means of one of said supports (4);
- a third electrode (LA) connected to the second central electrode (V2) by means of one of said supports (4);
- a fourth electrode (LL) connected to the second central electrode (V2) by means of one of said supports (4);
- a fifth electrode (V3) connected to the second central electrode (V2) by means of one of said supports (4);
- a sixth electrode (V4), a seventh electrode (V5) and an eighth electrode (V6) connected to the second central electrode (V2); in such an embodiment, the sixth, seventh and eighth electrode (V4, V5 and V6) are arranged in series downstream of the electrode (V3); in particular, the sixth electrode (V4) is connected to the fifth electrode (V3) by means of one of said supports (4), the seventh electrode (V5) is connected to the sixth electrode (V4) by means of one of said supports (4), and the eighth electrode (V6) is connected to the seventh electrode (V5) by means of one of said supports.

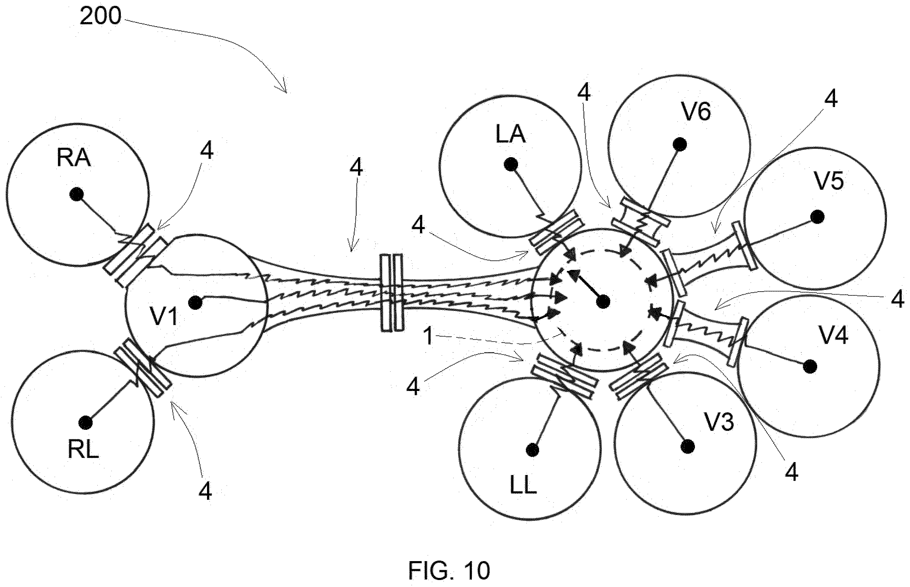

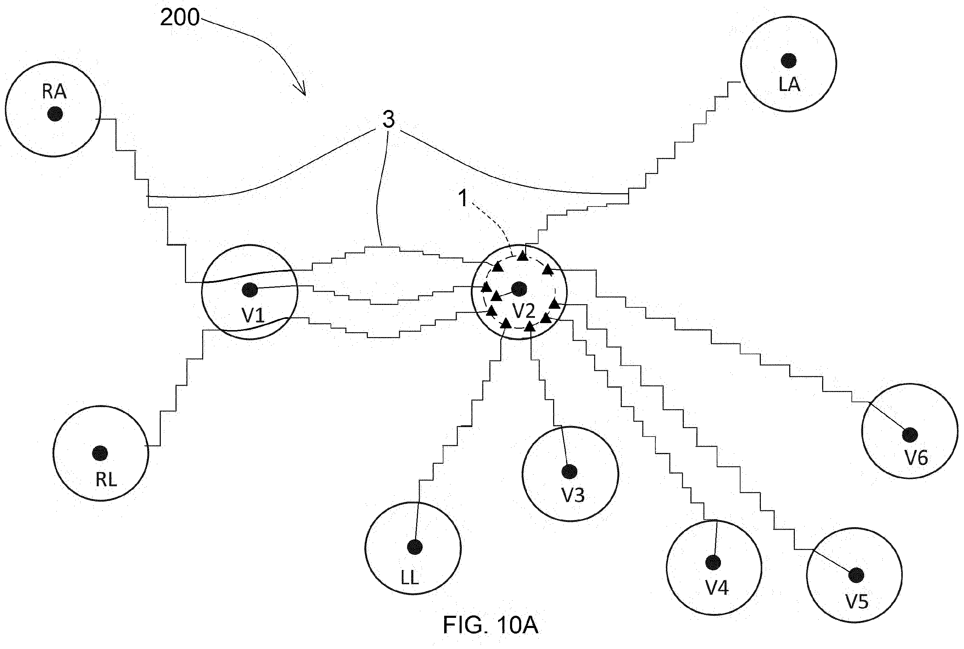

With particular reference to Figs. 10 and 10A, the electrode system (200) of the second embodiment comprises ten electrodes (2) and ten conductor cables (3).

In particular, the electrode system (200) comprises two central electrodes (V1, V2), namely a first central electrode (V1) and a second central electrode (V2). As shown in Fig. 10A, the connection terminal (1) is positioned in correspondence with the second central electrode (V2). It should be noted, however, that nothing would change for the achievement of the objectives pursued by the invention, if the connection terminal (1) were positioned only on the first central electrode (V1) or if the electrode system (200) comprised two connection terminals positioned on the two central electrodes (V1 and V2).

In the electrode system (200) of the second embodiment, said ten electrodes comprise eight external electrodes, namely:
- a first electrode (RA) connected to the first central electrode (V1) by means of one of said supports (4);
- a second electrode (RL) connected to the first central electrode (V1) by means of one of said supports (4);
- a third electrode (LA) connected to the second central electrode (V2) by means of one of said supports (4);
- a fourth electrode (LL) connected to the second central electrode (V2) by means of one of said supports (4);
- a fifth electrode (V3) connected to the second central electrode (V2) by means of one of said supports (4);
- a sixth electrode (V4), a seventh electrode (V5) and an eighth electrode (V6) connected to the second central electrode (V2); in this embodiment, the electrodes (V4, V5 and V6) are all connected to the second central electrode (V2), each one by means of one of said supports (4).

The electrode system (100) of the second embodiment is more reliable than the electrode system (100) of the first embodiment. In the electrode system (100) of the first embodiment version, if a user removes or separates the support (4) that connects the sixth electrode (V4) to the second central electrode (V2) incorrectly or too energetically, there is a possibility that all the connector cables supported by the support (4) will break or will be damaged, resulting in the loss of the signal detected by the sixth (V4), the seventh (V5), and the eighth (V6) electrode.

Instead, in the electrode system (200), the same accidental event would cause only the breakage or damage of the connector cable (3) associated with the sixth electrode (V4), resulting in the loss of the signal coming from only the sixth electrode (V4).

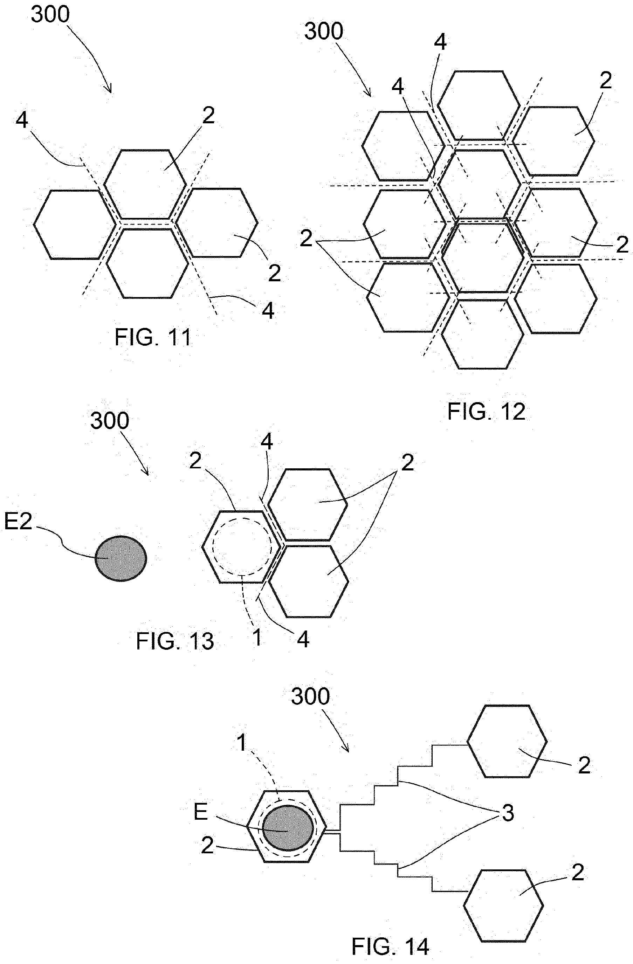

With reference to Figs. 11, 12, 13, 14, an additional embodiment of the electrode system (300) is shown, which can be defined as "honeycomb".

In such a case a plurality of electrodes (2) is provided, and when the electrode system (300) is in the space-saving configuration, said electrodes (2) are grouped in such a way as to form a honeycomb structure.

In Figs. 11 and 12, the supports (4) are indicated with dotted lines.

The electrodes (2) can be four (see Fig. 11) for fundamental and augmented derivations or can be ten (see Fig. 12) for fundamental, augmented, and precordial derivations.

Obviously, although the embodiment shown in Figs. 11 and 12 comprises four or ten electrodes, said electrodes can be of any number according to the requirements of the application in which the electrode system (300) is to be used.

By way of example, Figs. 13 and 14 show a system (300) with only three electrodes (2) in the compact configuration (Fig. 13) and in the open configuration with the supports removed or separated (Fig. 14).

Figs. 13 and 14 schematically show a device (E2) by means of a circle, comprising the signal receiving and processing system applied to the electrical connection terminal (1).

Therefore, the electrode system for the multiple acquisition of electrocardiographic derivations according to the invention makes the installation operation easy and practical, reducing or eliminating the presence of the electrical conductors connecting the individual electrodes to the electrocardiographic system.

Moreover, since the electrode system (100, 200, 300) provides for the use of removable or separable supports (4), the electrodes can be positioned in any point of the patient's body without the risk of detachment, since no elastic structural element is provided between adjacent electrodes, which would tend to bring the electrode system back to the non-compact configuration.

Moreover, the system is inexpensive to make.

Variations and equivalent modifications may be made to the present embodiment of the invention, within the reach of a skilled person in the art, which fall within the scope of the invention as expressed by the appended claims.

## Claims

1. An electrode system (100, 200, 300) for the multiple acquisition of electrocardiographic derivations comprising:
- a plurality of electrodes (2) of adhesive disposable type suitable for being applied on a user's body;
- at least one electrical connection terminal (1) for connecting the electrode system (100, 200, 300) with a signal receiving and processing system;
- a plurality of connector cables (3) connecting each electrode (2) to the at least one electrical connection terminal (1); and
- a plurality of supports (4) that connect said electrodes (2) to each other; each support (4) supporting at least one connector cable (3) that is rolled or bent in serpentine configuration; wherein each support (4) keeps two adjacent electrodes (2) close to each other in a space-saving configuration of the electrode system prior to use;
**characterized in that** each support (4) supporting the connector cable (3) is of a removable or separable type, in such a way that once the support (4) is removed or separated into a first half-shell (41) and a second half-shell (42) or into two separated portions (45a, 45b), thereby allowing to completely free the connector cables (3), the two electrodes (2) connected to the support (4) can be moved away from each other and the at least one connector cable (3) can be unrolled to use the system (100, 200, 300).

2. The electrode system (100, 200, 300) according to claim 1, wherein each electrode (2) comprises a connector (22); wherein for each electrode (2) the electrode system (100, 200, 300) comprises a coupling body (5) connected to at least one support (4) and comprising a connector (52) electrically connectable to the connector (22) of the electrode (2).

3. The electrode system (100, 200, 300) according to claim 2, wherein said connector (52) of the coupling body (5) is electrically connected to a connector cable (3).

4. The electrode system (100, 200, 300) according to any one of the preceding claims, wherein said at least one electrical connection terminal (1) is positioned in correspondence with at least one of said electrodes (2).

5. The electrode system (100, 200, 300) according to any one of the preceding claims, wherein said at least one electrical connection terminal (1) comprises an attachment pad (10) wherein said signal receiving and processing system is suitable for being attached.

6. The electrode system (100, 200, 300) according to any one of the preceding claims, wherein at least one of said supports (4) comprises a casing (40) that accommodates the connector cable (3) that is rolled or bent in serpentine configuration; wherein said casing (40) comprises:
- said first half-shell (41) and said second half-shell (42) separable from each other; and
- connection means (43; 44) that are interposed between the two half-shells (41 and 42) and that allow the two half-shells to be separated in such a way to open the casing (40).

7. The electrode system (100, 200, 300) according to any one of the preceding claims, wherein at least one of said supports (4) comprises a flat two-dimensional element (45) that extends between the two electrodes and has an adhesive surface whereon the connector cable (3) that is rolled or bent in serpentine configuration is attached.

8. The electrode system (100, 200, 300) according to claim 7, wherein said two-dimensional element (45) comprises said two portions that can be separated (45a, 45b) from each other by means of a pre-cut line (46).

9. The electrode system (100, 200) according to any one of the preceding claims, wherein said plurality of electrodes (2) comprises a first central electrode (V1) and a second central electrode (V2); wherein said at least one electrical connection terminal (1) comprises:
- a single electrical connection terminal (1) positioned in correspondence with the first central electrode (V1) or in correspondence with the second central electrode (V2); or
- two electrical connection terminals (1) positioned in correspondence with the first central electrode (V1) or in correspondence with the second central electrode (V2).

10. The electrode system (100, 200) according to claim 9, wherein said plurality of electrodes (2) comprises a plurality of external electrodes, namely:
- a first electrode (RA) connected to the first central electrode (V1) by means of one of said supports (4);
- a second electrode (RL) connected to the first central electrode (V1) by means of one of said supports (4);
- a third electrode (LA) connected to the second central electrode (V2) by means of one of said supports (4);
- a fourth electrode (LL) connected to the second central electrode (V2) by means of one of said supports (4);
- a fifth electrode (V3) connected to the second central electrode (V2) by means of one of said supports (4);
- a sixth electrode (V4), a seventh electrode (V5) and an eighth electrode (V6) connected to the second central electrode (V2).

11. The electrode system (200) according to claim 10, wherein :
- said sixth electrode (V4) is connected to said second central electrode (V2) by means of one of said supports (4);
- said seventh electrode (V5) is connected to said second central electrode (V2) by means of one of said supports (4);
- said eighth electrode (V6) is connected to the second central electrode (V2) by means of one of said supports (4).

12. The electrode system (300) according to any one of claims 1 to 8, wherein, when the electrode system (300) is in the space-saving configuration, said electrodes (2) are grouped in such a way as to form a honeycomb structure.

13. ECG measurement instrument comprising:
- said electrode system (100, 200, 300) according to any one of the preceding claims; and
- a signal receiving and processing system connected to the at least one terminal connection (1) of the electrode system (100, 200, 300).

14. The ECG measurement instrument according to claim 13, wherein said signal receiving and processing system comprises:
- a computing device (E1) disposed in an ECG apparatus (T) separate from the patient;
- a patient cable (C) electrically connected on one side to said at least one connection terminal (1) and on the other side to the computing device.

15. The ECG measurement instrument according to claim 13, wherein said signal receiving and processing system comprises at least one device (E2) directly connected and coupled to the connection terminal (1) of the electrode system (100, 200, 300).

## Patentansprüche

1. Elektrodensystem (100, 200, 300) für die mehrfache Erfassung von elektrokardiographischen Ableitungen, umfassend:
- eine Vielzahl von Elektroden (2) vom Klebe- und Einwegtyp, die dazu geeignet sind, am Körper eines Benutzers angebracht zu werden;
- mindestens eine elektrische Anschlussklemme (1) zum Verbinden des Elektrodensystems (100, 200, 300) mit einem Signalempfangs- und - verarbeitungssystem;
- eine Vielzahl von Anschlusskabeln (3), die jede Elektrode (2) mit mindestens einer Anschlussklemme (1) verbinden; und
- eine Vielzahl von Halterungen (4), die die Elektroden (2) miteinander verbinden; wobei jede Halterung (4) mindestens ein Anschlusskabel (3) hält, das serpentinenförmig aufgerollt oder gebogen ist; wobei jede Halterung (4) zwei aneinander angrenzende Elektroden (2) vor dem Gebrauch in einer platzsparenden Anordnung des Elektrodensystems dicht nebeneinanderhält;
- **dadurch gekennzeichnet, dass** jede Halterung (4), die das Anschlusskabel (3) hält, vom abnehmbaren oder teilbaren Typ ist, derart, dass, wenn die Halterung (4) abgenommen oder in eine erste Halbschale (41) und eine zweite Halbschale (42) oder in zwei getrennte Abschnitte (45a, 45b) geteilt wird und es dadurch ermöglicht, dass die Anschlusskabel (3) vollständig frei kommen, die beiden Elektroden (2), die mit der Halterung (4) verbunden sind, voneinander weg bewegt können und das mindestens eine Anschlusskabel (3) abgerollt werden kann, um das System (100, 200, 300) zu benutzen.

2. Elektrodensystem (100, 200, 300) nach Anspruch 1, wobei jede Elektrode (2) einen Steckeranschluss (22) umfasst; wobei jede Elektrode (2) des Elektrodensystems (100, 200, 300) einen Kupplungskörper (5) umfasst, der mit mindestens einer Halterung (4) verbunden ist und einen Steckeranschluss (52) umfasst, der mit dem Steckeranschluss (22) der Elektrode (2) elektrisch verbindbar ist.

3. Elektrodensystem (100, 200, 300) nach Anspruch 2, wobei der Steckeranschluss (52) des Kupplungskörpers (5) mit dem Anschlusskabel (3) elektrisch verbunden ist.

4. Elektrodensystem (100, 200, 300) nach einem der vorstehenden Ansprüche, wobei die mindestens eine elektrische Anschlussklemme (1) in Übereinstimmung mit mindestens einer der Elektroden (2) positioniert ist.

5. Elektrodensystem (100, 200, 300) nach einem der vorstehenden Ansprüche, wobei die mindestens eine elektrische Anschlussklemme (1) ein Haftpolster (10) umfasst, in dem das Signalempfangs- und - verarbeitungssystem geeignet ist, angebracht zu werden.

6. Elektrodensystem (100, 200, 300) nach einem der vorstehenden Ansprüche, wobei mindestens eine der Halterungen (4) ein Gehäuse (40) umfasst, in dem das Anschlusskabel (3) untergebracht ist, das serpentinenförmigen aufgerollt oder gebogen ist; wobei das Gehäuse (40) umfasst:
- eine erste Halbschale (41) und eine zweite Halbschale (42), die voneinander trennbar sind; und
- Verbindungsmittel (43; 44), die zwischen den beiden Halbschalen (41 und 42) angeordnet sind und es den beiden Halbschalen erlauben, so getrennt zu werden, dass sich das Gehäuse (40) öffnet.

7. Elektrodensystem (100, 200, 300) nach einem der vorstehenden Ansprüche, wobei mindestens eine der Halterungen (4) ein flaches zweidimensionales Element (45) umfasst, das sich zwischen den beiden Elektroden erstreckt und eine Klebefläche aufweist, auf der das Anschlusskabel (3), das serpentinenförmig aufgerollt oder gebogen ist, angebracht ist.

8. Elektrodensystem (100, 200, 300) nach Anspruch 7, wobei das zweidimensionale Element (45) zwei Abschnitte umfasst, die mittels einer vorgeschnittenen Linie (46) voneinander getrennt (45a, 45b) werden können.

9. Elektrodensystem (100, 200, 300) nach einem der vorstehenden Ansprüche, wobei die Vielzahl von Elektroden (2) eine erste zentrale Elektrode (V1) und eine zweite zentrale Elektrode (V2) umfassen; wobei die mindestens eine elektrische Anschlussklemme (1) Folgendes umfasst:
- eine einzelne elektrische Anschlussklemme (1), die in Übereinstimmung mit der ersten zentralen Elektrode (V1) oder in Übereinstimmung mit der zweiten zentralen Elektrode (V2) positioniert ist; oder
- zwei elektrische Anschlussklemmen (1), die in Übereinstimmung mit der ersten zentralen Elektrode (V1) oder in Übereinstimmung mit der zweiten zentralen Elektrode (V2) positioniert ist.

10. Elektrodensystem (100, 200, 300) nach Anspruch 9, wobei die Vielzahl von Elektroden (2) eine Vielzahl von Außenelektroden umfasst, und zwar:
- eine erste Elektrode (RA), die mit der ersten zentralen Elektrode (V1) mittels einer der Halterungen (4) verbunden ist;
- eine zweite Elektrode (RL), die mit der ersten zentralen Elektrode (V1) mittels einer der Halterungen (4) verbunden ist;
- eine dritte Elektrode (LA), die mit der zweiten zentralen Elektrode (V2) mittels einer der Halterungen (4) verbunden ist;
- eine vierte Elektrode (LL), die mit der zweiten zentralen Elektrode (V2) mittels einer der Halterungen (4) verbunden ist;
- eine fünfte Elektrode (V3), die mit der zweiten zentralen Elektrode (V2) mittels einer der Halterungen (4) verbunden ist;
- eine sechste Elektrode (V4), eine siebte Elektrode (V5) und eine achte Elektrode (V6), die mit der zweiten zentralen Elektrode (V2) verbunden sind.

11. Elektrodensystem (200) nach Anspruch 10, wobei:
- die sechste Elektrode (V4) mit der zweiten zentralen Elektrode (V2) mittels einer der Halterungen (4) verbunden ist;
- die siebte Elektrode (V6) mit der zweiten zentralen Elektrode (V2) mittels einer der Halterungen (4) verbunden ist;
- die achte Elektrode (V6) mit der zweiten zentralen Elektrode (V2) mittels einer der Halterungen (4) verbunden ist.

12. Elektrodensystem (300) nach einem der Ansprüche von 1 bis 8, wobei, wenn das Elektrodensystem (300) sich in der platzsparenden Anordnung befindet, die Elektroden (2) derart gruppiert sind, dass sie eine Wabenstruktur bilden.

13. EKG-Messgerät, umfassend:
- das Elektrodensystem (100, 200, 300) nach einem der vorstehenden Ansprüche; und
- ein Signalempfangs- und -verarbeitungssystem, das mit der mindestens einen Anschlussklemme (1) des Elektrodensystems (100, 200, 300) verbunden ist.

14. EKG-Messgerät nach Anspruch 13, wobei das Signalempfangs- und - verarbeitungssystem Folgendes umfasst:
- eine Datenverarbeitungseinrichtung (E1), die in einem EKG-Gerät (T) getrennt vom Patienten angeordnet ist;
- ein Patientenkabel (C), das auf einer Seite an die mindestens eine Anschlussklemme (1) und auf der anderen Seite an die Datenverarbeitungseinrichtung elektrisch anschlossen ist.

15. EKG-Messgerät nach Anspruch 13, wobei das Signalempfangs- und - verarbeitungssystem mindestens eine Vorrichtung (E2) umfasst, die direkt an die Anschlussklemme (1) des Elektrodensystems (100, 200, 300) angeschlossen und gekoppelt ist.

## Revendications

1. Système d'électrodes (100, 200, 300) pour l'acquisition multiple de dérivations électrocardiographiques comprenant :
- une pluralité d'électrodes (2) adhésives jetables aptes à être appliquées sur le corps d'un patient ;
- au moins une borne de connexion électrique (1) pour connecter le système d'électrodes (100, 200, 300) à un système de réception et de traitement de signal ;
- une pluralité de câbles connecteurs (3) connectant chaque électrode (2) à au moins une borne de connexion électrique (1) ; et
- une pluralité de supports (4) qui connectent lesdites électrodes (2) les unes aux autres ; chaque support (4) soutenant au moins un câble connecteur (3) qui est enroulé ou plié en configuration zigzag ; où chaque support (4) maintient deux électrodes adjacentes (2) proches l'une de l'autre dans une configuration compacte du système d' électrodes avant usage ;
**caractérisé en ce que** chaque support (4) qui soutient le câble connecteur (3) est du type amovible ou séparable, de sorte que lorsque le support (4) est retiré ou séparé en une première demi-coquille (41), puis en une deuxième demi-coquille (42) ou en deux portions séparées (45a, 45b), permettant ainsi de libérer complètement les câbles connecteurs (3), les deux électrodes (2) connectées au support (4) peuvent être déplacées en les éloignant l'une de l'autre et le susdit au moins un câble connecteur (3) peut être déroulé pour utiliser le système (100, 200, 300).

2. Système d'électrodes (100, 200, 300) selon la revendication 1, où chaque électrode (2) comprend un connecteur (22) ; où, pour chaque électrode (2), le système d'électrodes (100, 200, 300) comprend un corps de couplage (5) connecté à au moins un support (4) et comprenant un connecteur (52) électriquement connectable au connecteur (22) de l'électrode (2).

3. Système d'électrodes (100, 200, 300) selon la revendication 2, où ledit connecteur (52) du corps de couplage (5) est connecté électriquement à un câble connecteur (3).

4. Système d'électrodes (100, 200, 300) selon l'une quelconque des revendications précédentes, où ladite au moins une borne de connexion électrique (1) est positionnée en correspondance d'au moins une desdites électrodes (2).

5. Système d'électrodes (100, 200, 300) selon l'une quelconque des revendications précédentes, où ladite au moins une borne de connexion électrique (1) comprend un plot de fixation (10) sur lequel ledit système de réception et de traitement de signal est adapté pour être fixé.

6. Système d'électrodes (100, 200, 300) selon l'une quelconque des revendications précédentes, où au moins un des lesdits supports (4) comprend un boîtier (40) qui reçoit le câble connecteur (3) qui est enroulé ou plié en configuration zigzag ; où ledit boîtier (40) comprend :
- ladite première demi-coquille (41) et ladite deuxième demi-coquille (42) séparables l'une de l'autre ; et
- des moyens de connexion (43; 44) qui sont interposés entre les deux demi-coquilles (41 et 42) et qui permettent de séparer les deux demi-coquilles de manière à ouvrir le boîtier (40).

7. Système d'électrodes (100, 200, 300) selon l'une quelconque des revendications précédentes, où au moins un des lesdits supports (4) comprend un élément bidimensionnel plat (45) qui se déploie entre les deux électrodes et a une surface adhésive sur laquelle est attaché le câble connecteur (3) qui est enroulé ou plié en configuration zigzag.

8. Système d'électrodes (100, 200, 300) selon la revendication 7, où ledit élément bidimensionnel (45) comprend lesdites deux portions qui peuvent être séparées (45a, 45b) l'une de l'autre au moyen d'une ligne de prédécoupe (46).

9. Système d'électrodes (100, 200) selon l'une quelconque des revendications précédentes, où ladite pluralité d'électrodes (2) comprend une première électrode centrale (V1) et une deuxième électrode centrale (V2) ; où ladite au moins une borne de connexion électrique (1) comprend :
- une seule borne de connexion électrique (1) positionnée en correspondance de la première électrode centrale (V1) ou en correspondance de la seconde électrode centrale (V2) ; ou
- deux bornes de connexion électrique (1) positionnées en correspondance de la première électrode centrale (V1) ou en correspondance de la deuxième électrode centrale (V2).

10. Système d'électrodes (100, 200) selon la revendication 9, où ladite pluralité d'électrodes (2) comprend une pluralité d'électrodes externes, notamment :
- une première électrode (RA) connectée à la première électrode centrale (V1) moyennant l'un desdits supports (4) ;
- une deuxième électrode (RL) connectée à la première électrode centrale (V1) moyennant l'un desdits supports (4) ;
- une troisième électrode (LA) connectée à la deuxième électrode centrale (V2) moyennant l'un desdits supports (4) ;
- une quatrième électrode (LL) connectée à la deuxième électrode centrale (V2) moyennant l'un desdits supports (4) ;
- une cinquième électrode (V3) connectée à la deuxième électrode centrale (V2) moyennant l'un desdits supports (4) ;
- une sixième électrode (V4), une septième électrode (V5) et une huitième électrode (V6) connectées à la deuxième électrode centrale (V2).

11. Système d'électrodes (200) selon la revendication 10, où :
- ladite sixième électrode (V4) est connectée à ladite deuxième électrode centrale (V2) moyennant l'un desdits supports (4) ;
- ladite septième électrode (V5) est connectée à ladite deuxième électrode centrale (V2) moyennant l'un desdits supports (4) ;
- ladite huitième électrode (V6) est connectée à la deuxième électrode centrale (V2) moyennant l'un desdits supports (4).

12. Système d'électrodes (300) selon l'une quelconque des revendications de 1 à 8, où, lorsque le système d'électrodes (300) est dans sa configuration compacte, lesdites électrodes (2) sont regroupées d'une façon telle qu'elles forment une structure en nid d'abeille.

13. instrument de mesure ECG comprenant :
- ledit système d'électrodes (100, 200, 300) selon l'une quelconque des revendications précédentes ; et
- un système de réception et de traitement de signal connecté à au moins une borne de connexion (1) du système d'électrodes (100, 200, 300).

14. instrument de mesure ECG selon la revendication 13, où ledit système de réception et de traitement de signal comprend :
- un dispositif informatique (E1) disposé dans un appareil ECG (T) séparé du patient ;
- un câble patient (C) connecté électriquement d'un côté à la susdite au moins une borne de connexion (1) et de l'autre côté au dispositif informatique.

15. instrument de mesure ECG selon la revendication 13, où ledit système de réception et de traitement de signal comprend au moins un dispositif (E2) directement connecté et accouplé à la borne de connexion (1) du système d'électrodes (100, 200, 300).
